# EUROPÄISCHE PATENTSCHRIFT

(11) **EP 2 547 377 B1**
(45) Veröffentlichungstag und Bekanntmachung des Hinweises auf die Patenterteilung: **02.05.2018**
(21) Anmeldenummer: 11708413.7
(22) Anmeldetag: 14.03.2011
(51) Int. Cl.: A61M 1/16

(54) **SYSTEM ZUR DURCHFÜHRUNG EINER BLUTBEHANDLUNG**
SYSTEM FOR CARRYING OUT A BLOOD TREATMENT
SYSTÈME POUR L'EXÉCUTION D'UN TRAITEMENT DU SANG

(30) Priorität: 15.03.2010 DE 102010011465
(43) Veröffentlichungstag der Anmeldung: 23.01.2013
(73) Patentinhaber: Fresenius Medical Care Deutschland GmbH, 61352 Bad Homburg (DE)
(72) Erfinder: POHLMEIER, Robert, 61350 Bad Homburg (DE); HERRENBAUER, Michael, 61267 Neu-Anspach (DE); GÖMPEL-KLEIN, Patricia, 61273 Wehrheim (DE); KRAUSE, Alfred, 61273 Wehrheim (DE); WEHMEYER, Wolfgang, 72076 Tübingen (DE)
(74) Vertreter: Herrmann, Uwe
(86) Internationale Anmeldenummer: PCT/EP2011/001261
(87) Internationale Veröffentlichungsnummer: WO 2011/113572

(56) Entgegenhaltungen:
- WO-A1-99/00154
- WO-A2-01/64262
- DE-A1-102007 009 269
- DE-U1- 9 302 790
- US-A- 4 229 299
- US-A- 4 765 888
- US-A1- 2005 187 529

## Beschreibung

Die vorliegende Erfindung betrifft ein System zur Durchführung einer Blutbehandlung, insbesondere einer Dialysebehandlung, wobei das System wenigstens ein Blutbehandlungsgerät, insbesondere ein Dialysegerät, enthält.

Aus dem Stand der Technik sind Dialysegeräte bekannt, bei denen die Dialysierflüssigkeit nicht während einer Behandlung hergestellt wird, sondern bei denen die gesamte für eine Dialysebehandlung erforderliche Menge an Dialysierflüssigkeit vor der Behandlung in einem Tank bereitgestellt wird. Derartige Dialysegeräte werden auch als "Batch-Type"-Dialysegeräte bezeichnet.

Aus dem Stand der Technik gemäß der WO 2008/104367 A2 ist es des weiteren bekannt, diese Geräte mit einer mobilen Einheit zu verbinden, in der die Dialysierflüssigkeit zubereitet wird und von der die zubereitete Dialysierflüssigkeit in den Tank des Dialysegerätes eingefüllt wird.

Bei dem aus der WO 2008/104367 A2 bekannten System wird somit die zubereitete Dialysierflüssigkeit von dem Tank der mobilen Einheit in den Tank des Dialysegerätes überführt.

Der vorliegenden Erfindung liegt die Aufgabe zugrunde, ein System der eingangs genannten Art dahingehend weiterzubilden, dass das System einfach handhabbar ist und einen Wechsel der Behandlungsflüssigkeit ermöglicht, während der Patient mit der Behandlungseinheit des Systems verbunden bleibt.

Diese Aufgabe wird durch ein System mit den Merkmalen des Anspruchs 1 gelöst. Danach ist vorgesehen, dass der Tank, aus dem während der Blutbehandlung Behandlungsflüssigkeit, insbesondere Dialysierflüssigkeit, entnommen wird, Bestandteil wenigstens einer mobilen Vorrichtung ist, die mit dem Blutbehandlungsgerät derart verbindbar ist, dass wenigstens eine Fluidverbindung zwischen dem Tank und dem Blutbehandlungsgerät herstellbar ist. Erfindungsgemäß ist somit vorgesehen, dass der Tank, aus dem während der Blutbehandlung bzw. Dialysebehandlung die Dialysierflüssigkeit entnommen wird, Bestandteil eines mobilen, das heißt durch einen Nutzer bewegbaren Gerätes ist, das vor der Behandlung mit dem Blutbehandlungsgerät dahingehend verbunden wird, dass eine Fluidverbindung zwischen dem Tank und dem Blutbehandlungsgerät besteht, so dass die Behandlungsflüssigkeit dem Blutbehandlungsgerät zugeführt werden kann.

Denkbar ist es, dass das Blutbehandlungsgerät selbst keinerlei Tank aufweist, aus dem während der der Blutbehandlung Behandlungsflüssigkeit, insbesondere Dialysierflüssigkeit, entnommen wird und/oder in den während der Durchführung der Blutbehandlung verbrauchte Behandlungsflüssigkeit, insbesondere verbrauchte Dialysierflüssigkeit, eingefüllt wird.

Bei einer solchen Ausführungsform der Erfindung ist jedoch nicht ausgeschlossen, dass beispielsweise ein oder mehrere Behälter für Antikoagulantien oder sonstige Zusatzstoffe an dem Blutbehandlungsgerät selbst angeordnet sind.

Auch ist eine Ausführungsform denkbar und von der Erfindung mit umfaßt, bei der an dem Blutbehandlungsgerät selbst ein vergleichsweise kleiner Zwischenspeicher, beispielsweise ein Behältnis mit einem Volumen von bis zu 1 I, angeordnet ist. Dies ermöglicht beispielsweise die Blutrückgabe, während kein Batch angeschlossen ist.

Das Blutbehandlungsgerät selbst kann somit vergleichsweise einfach ausgeführt sein, da es in diesem Fall keinen eigenen Tank aufweist, der zur Aufnahme von Dialysierflüssigkeit dient. Der Vorteil dieser Ausführungsform liegt in der Flexibilität und in der Möglichkeit eines Tankwechsels.

In weiterer Ausgestaltung der Erfindung weist das System Mittel auf, die derart ausgeführt sind, dass sie nach dem Bewegen der mobilen Vorrichtung in das Blutbehandlungsgerät in eine vorbestimmte Position automatisch oder auf Aufforderung wenigstens eine Fluidverbindung zwischen dem Tank und dem Blutbehandlungsgerät herstellen.

Grundsätzlich ist es denkbar, dass das Blutbehandlungsgerät automatisch das Vorhandensein der mobilen Vorrichtung erkennt und dann entweder automatisch oder auf Aufforderung durch den Nutzer wenigstens eine Fluidverbindung zwischen dem Tank und dem Blutbehandlungsgerät herstellt, so dass die Dialysierflüssigkeit dem Blutbehandlungsgerät bzw. dessen Mitteln zur Durchführung einer Blutbehandlung zugeführt werden kann.

Es können Mittel zur Herstellung einer Fluidverbindung zwischen dem Tank der mobilen Vorrichtung einerseits und dem Blutbehandlungsgerät andererseits vorgesehen sein. Diese können Schläuche und/oder Verbindungselemente umfassen, durch die die Behandlungsflüssigkeit nach der Konnektion hindurchströmt. Die genannten Mittel können in oder an dem Blutbehandlungsgerät und/oder in oder an der mobilen Vorrichtung angeordnet sein.

Grundsätzlich ist es denkbar, dass das Blutbehandlungsgerät automatisch erkennt, wenn die mobile Vorrichtung in eine vorbestimmte und vorgesehene Endposition bewegt wurde und dann automatisch eine Fluidverbindung und/oder eine Datenverbindung herstellt oder zumindest dem Nutzer die Möglichkeit gibt, auf Aufforderung entsprechende Verbindungen herzustellen.

Sofern es sich um eine Fluidverbindung handelt, ist diese derart auszuführen, dass die mikrobielle Kontaminationsgefahr (entsprechend dem Stand der Technik) minimiert wird, wobei die Fluidverbindung zum Beispiel durch entsprechende Verbindungselemente zwischen dem wenigstens einen Tank einerseits und dem Blutbehandlungsgerät andererseits hergestellt wird.

In weiterer Ausgestaltung der Erfindung ist vorgesehen, dass das Blutbehandlungsgerät eine Aufnahme, vorzugsweise eine Ausnehmung oder einen Raum zur Aufnahme der mobilen Vorrichtung aufweist. Um eine Dialysebehandlung beginnen zu können, wird die mobile Vorrichtung in diese Ausnehmung bzw. in den dafür vorgesehenen Raum eingeführt und konnektiert. Anschließend kann die Blutbehandlung durch das Blutbehandlungsgerät bzw. durch dessen Mittel zur Durchführung einer externen Blutbehandlung aufgenommen werden.

Für die sichere Integration der mobilen Vorrichtung in das Blutbehandlungsgerät ist es von Vorteil, dass die mobile Vorrichtung gut manövrierbar ist und mit dem Blutbehandlungsgerät in geeigneter Weiseverbunden, vorzugsweise verrastet wird. Die gute Manövrierbarkeit der mobilen Vorrichtung kann beispielsweise durch Rollen der mobilen Vorrichtung bzw. durch ein eigenes Rollensystem erreicht werden. Vorzugsweise sind die Rollen bzw. das Rollensystem derart ausgeführt, dass ein sicherer Geradeauslauf der mobilen Vorrichtung gewährleistet wird, da die mobile Vorrichtung vorzugsweise im Geradeauslauf in das Blutbehandlungsgerät eingeschoben werden muss.

Zugleich sollte das Rollensystem bzw. die Rollen derart ausgeführt sein, dass eine vergleichsweise einfache Lenkbarkeit der mobilen Vorrichtung gewährleistet ist.

Diese kann im befüllten Zustand ein Gewicht in der Größenordnung im Bereich von ca. 15 kg bis 150 kg, insbesondere weniger als 120 kg und beispielsweise 80 kg aufweisen.

In bevorzugter Ausgestaltung der Erfindung ist des weiteren vorgesehen, dass die Rollen der mobilen Vorrichtung möglichst wenig seitlich herausragen bzw. eine möglichst geringe "Spurweite" beanspruchen, flach und möglichst nicht oder wenig ausschwenkend konstruiert sind, damit ein Verhaken der Rollen mit dem Blutbehandlungsgerät möglichst vermieden wird. Vorzugsweise sind die Rollen bzw. Räder der mobilen Vorrichtung gemäß der DE 19 39 932 A1 ausgeführt, auf die insoweit Bezug genommen wird. Demnach können die Rollen bzw. Räder aus einem rotierbar angeordneten Nabenkörper bestehen, der auf seinem Umfang mehrere über den Nabenkörperumfang hinaus ragende drehbare Rollen aufweist, deren Drehachsen in Richtung der Tangenten an den Nabenkörperumfang verlaufen. Durch diese Ausgestaltung ist es möglich, die mobile Vorrichtung auch im belasteten Zustand ohne weiteres in zueinander senkrechten Richtungen zu bewegen.

Hinsichtlich weiterer denkbarer Ausgestaltungen der Rollen bzw. Räder wird auf die DE 19 39 932 A1 Bezug genommen, die insoweit zum Offenbarungsgehalt der vorliegenden Erfindung gemacht wird.

Der Tank kann als starres Gefäß oder auch als zumindest teilweise flexibler Beutel ausgeführt sein.

Vorzugsweise ist vorgesehen, dass der Tank als Einmalartikel ausgeführt ist. Der Tank, der als Beutel ausgeführt sein kann, wird in bevorzugter Ausgestaltung der Erfindung für jede neue Befüllung ausgetauscht. Dadurch kann die Reinigung zwischen den Füllungen entfallen.

Vorteilhaft ist es, wenn die mobile Vorrichtung derart ausgeführt ist, dass sie den Tank, insbesondere den Beutel mechanisch stützt. Dies hat den Vorteil, dass der Tank bzw. der Beutel selbst eine geringere Festigkeit aufweisen muss, als wenn eine mechanische Unterstützung durch die mobile Vorrichtung nicht vorhanden wäre. In weiterer bevorzugter Ausgestaltung der Erfindung ist vorgesehen, dass die Vorrichtung gasdicht ausgeführt ist. Dies bedeutet, dass die Vorrichtung selbst als Gasbarriere dient, um den Verlust von CO₂ zu verhindern. Diese Ausgestaltung der Erfindung ermöglicht die Verwendung eines vergleichsweise günstigen Materials des Tanks (der beispielsweise als Beutel ausgeführt sein kann), da an dessen Gasbarriereeigenschaften entsprechend geringere Anforderungen zu stellen sind.

Es ist vorgesehen, dass der Tank als Mehrkammertank, insbesondere als Mehrkammerbeutel ausgeführt ist, von dem wenigstens eine Kammer zur Aufnahme von Behandlungsflüssigkeit dient und von dem wenigstens eine Kammer zur Aufnahme verbrauchter Behandlungsflüssigkeit dient. Denkbar ist es, dass die mobile Vorrichtung Mittel zum Ableiten verbrauchter Behandlungsflüssigkeit in einen Abfluß oder in eine sonstige Aufnahmeeinheit aufweist.

Ist der Tank als Mehrkammerbeutel vorgesehen, bringt dies den Vorteil mit sich, dass das Beutelvolumen insgesamt während der Behandlung nicht oder nur unwesentlich Veränderungen unterliegt. Denkbar ist es, dass das Volumen an Dialysierflüssigkeit, die dem Behandlungsgerät zugeführt wird, durch das Volumen von verbrauchter Dialysierflüssigkeit und gegebenenfalls zusätzlich von Ultrafiltrat ersetzt wird.

Ein weiterer Vorteil besteht in der internen Wärmerückgewinnung: das gebrauchte Dialysat gibt Wärme an das frische Dialysat ab, was in einem reduzierten Heizungsbedarf resultiert. Umschließt der Tank bzw. Beutel für das gebrauchte Dialysat den Tank bzw. Beutel für das frische Dialysat (Bag in Bag Konzept), ergibt sich als weiterer Vorteile eine gute Wärmeisolation für das frische Dialysat.

Alternativ zu einer solchen Vorgehensweise ist es selbstverständlich auch möglich, einen weiteren Tank bzw. Beutel zu verwenden, in dem verbrauchte Dialysierflüssigkeit und gegebenenfalls Ultrafiltrat aufgenommen wird. Schließlich ist es auch möglich, eine direkte Abflussleitung vorzusehen, durch die verbrauchte Behandlungsflüssigkeit und gegebenenfalls Ultrafiltrat mittels der mobilen Vorrichtung in einen Abfluß bzw. in eine sonstige Aufnahmeeinheit geleitet wird.

In weiterer Ausgestaltung der Erfindung ist vorgesehen, dass das System des Weiteren wenigstens eine Befülleinheit aufweist, mittels derer der Tank der mobilen Vorrichtung mit Behandlungsflüssigkeit befüllbar ist.

Die Befülleinheit kann Mittel aufweisen, mittels derer sie aus mehreren Komponenten, vorzugsweise aus einem oder mehreren Konzentraten und Wasser die Behandlungsflüssigkeit herstellt.

Denkbar ist es auch, dass die Befülleinheit auch nur Wasser in geeigneter Qualität (Reinheit, Temperatur und Menge) zur Verfügung stellt. Dabei kann vorgesehen sein, dass das Konzentrat bereits in dem Tank bzw. Beutel vorab vorliegt.

Die Befülleinheit kann ferner Mittel zur Entleerung der gebrauchten Lösung aufweisen.

Zu diesem Zwecke kann die Befülleinheit mit einer RO-Wasserversorgungseinheit in Verbindung stehen oder eine solche Einheit aufweisen.

Wie oben ausgeführt, ist es denkbar, dass die mobile Vorrichtung mit dem Blutbehandlungsgerät in geeigneter Weise verbunden wird. Dabei ist darauf zu achten, dass ein Wegrollen der mobilen Vorrichtung aus ihrer Stationsposition heraus verhindert wird. Denkbar ist es, die mobile Vorrichtung mit einem Führungselement, beispielsweise mit einer Führungszunge des Blutbehandlungsgerätes zu verbinden bzw. zu verrasten, wenn die mobile Vorrichtung in ihrer endgültigen Position steht. Dieses Führungselement bzw. die Führungszunge bietet darüber hinaus eine gute Möglichkeit, die vergleichsweise schwere, gefüllte Vorrichtung in das Blutbehandlungsgerät einzuführen, ohne dass sich beim Einschieben die mobile Vorrichtung und das Blutbehandlungsgerät verhaken können. Eine derartige Führung kann an der mobilen Vorrichtung und/oder an dem Blutbehandlungsgerät angeordnet sein.

Um das Einrasten und Lösen der mobilen Vorrichtung mit geringem Kraftaufwand zu ermöglichen, kann eine Kugel vorgesehen sein, die beispielsweise auf der Unterseite der mobilen Vorrichtung angebracht ist und die in eine Ausnehmung des Blutbehandlungsgerätes, beispielsweise der Führungszunge eingreifen kann.

Die vorliegende Erfindung betrifft des weiteren ein Verfahren zum Versorgen des Blutbehandlungsgerätes, insbesondere eines Dialysegerätes, mit der Behandlungsflüssigkeit, insbesondere mit der Dialysierflüssigkeit, die während der Durchführung der Blutbehandlung aus dem Tank entnommen wird. Das Verfahren ist dadurch gekennzeichnet, dass der Tank Bestandteil einer mobilen Vorrichtung ist, die zur Durchführung der Blutbehandlung mit dem Blutbehandlungsgerät derart verbunden wird, dass eine Fluidverbindung zwischen dem Tank und dem Blutbehandlungsgerät besteht.

Der Begriff "Tank" ist weit zu fassen und umfaßt beispielsweise auch Fertigarzneimittel, wie beispielsweise HF-Lösungen.

Das Verfahren kann des Weiteren den Schritt umfassen, dass verbrauchte Behandlungsflüssigkeit, insbesondere verbrauchte Dialysierflüssigkeit dem bzw. einem Tank der mobilen Vorrichtung zugeführt wird oder mittels der mobilen Vorrichtung einem Ablauf oder einer sonstigen Aufnahmeeinheit zugeführt wird.

In weiterer Ausgestaltung der Erfindung ist vorgesehen, dass die mobile Vorrichtung zunächst zu einer Befülleinheit bewegt wird, in der der Tank der mobilen Vorrichtung mit Behandlungsflüssigkeit, insbesondere mit frischer Dialysierflüssigkeit, befüllt wird.

Das Verfahren kann derart durchgeführt werden, dass dem wenigstens einen Tank gleichzeitig oder zeitversetzt Behandlungsflüssigkeit entnommen und gebrauchte Behandlungsflüssigkeit zugeführt wird. Grundsätzlich ist von der Erfindung auch der Fall umfasst, dass unterschiedliche Tanks bzw. Beutel eingesetzt werden, die zum einen die frische Behandlungsflüssigkeit bevorraten und zum anderen die verbrauchte Behandlungsflüssigkeit aufnehmen.

Das Verfahren kann des weiteren den Schritt umfassen, dass nach dem Bewegen der mobilen Vorrichtung zu dem Blutbehandlungsgerät in eine vorgegebene Position automatisch oder auf Aufforderung wenigstens eine Fluidverbindung zwischen dem Tank und dem Blutbehandlungsgerät hergestellt wird. Entsprechendes kann auch für eine Datenverbindung gelten, die dazu dient, Daten von der mobilen Vorrichtung an das Blutbehandlungsgerät und/oder umgekehrt zu übertragen.

Die mobile Vorrichtung kann zu der Befülleinheit verfahren werden und gebrauchte Behandlungsflüssigkeit, insbesondere gebrauchte Dialysierflüssigkeit kann aus dem Tank der mobilen Vorrichtung der Befülleinheit zugeführt werden. So ist es denkbar, dass die Befülleinheit nicht nur zum Befüllen des Tanks der mobilen Vorrichtung mit Behandlungsflüssigkeit dient, sondern auch dazu, gebrauchte Behandlungsflüssigkeit aus dem Tank der mobilen Vorrichtung abzuziehen. Aus hygienischen Gründen sind getrennte Fluidkreisläufe besonders vorteilhaft. Es kann auch eine separate Entleereinheit zum Einsatz kommen.

Die vorliegende Offenbarung betrifft des weiteren ein Blutbehandlungsgerät, insbesondere ein Dialysegerät mit Mitteln zur Durchführung einer Blutbehandlung, vorzugsweise einer externen Blutbehandlung und insbesondere einer Dialysebehandlung. Das Gerät ist dadurch gekennzeichnet, dass es keinen Tank aufweist, in dem eine zur Durchführung der Blutbehandlung benötigte Behandlungsflüssigkeit, insbesondere Dialysierflüssigkeit, vorliegt. Vielmehr ist vorgesehen, dass das Blutbehandlungsgerät wenigstens einen Anschluß aufweist, mittels dessen das Blutbehandlungsgerät mit wenigstens einem Tank verbindbar ist, in dem die Behandlungsflüssigkeit vorliegt, und durch den die Behandlungsflüssigkeit dem Blutbehandlungsgerät während der Blutbehandlung zuführbar ist.

Das Blutbehandlungsgerät kann wenigstens eine Aufnahme aufweisen, in die eine mobile Vorrichtung, vorzugsweise eine erfindungsgemäße mobile Vorrichtung, aufnehmbar ist, die den wenigstens einen Tank aufweist.

Weitere Einzelheiten und Vorteile der Erfindung werden anhand eines in der Zeichnung dargestellten Ausführungsbeispiels näher erläutert. Es zeigen:
- Figur 1:: eine schematische Darstellung eines erfindungsgemäßen Systems mit Dialysegerät, mobiler Vorrichtung und Befülleinheit,
- Figur 2:: eine schematische Ansicht der Befüllung des Tanks der mobilen Vorrichtung durch die Befülleinheit und der Ankopplung der mobilen Vorrichtung an das Blutbehandlungsgerät,
- Figur 3:: eine schematische Ansicht der mobilen Vorrichtung mit einem Zweikammerbeutel, wobei die Kammer zur Aufnahme der unverbrauchten Dialysierflüssigkeit gefüllt ist,
- Figur 4:: eine schematische Ansicht der mobilen Vorrichtung mit einem Zweikammerbeutel, wobei die Kammer zur Aufnahme der verbrauchten Dialysierflüssigkeit gefüllt ist und
- Figur 5:: eine schematische Ansicht der Befülleinheit mit der mobilen Vorrichtung, wobei die Befülleinheit auch als Einheit zur Entleerung des Tanks der mobilen Vorrichtung dient.

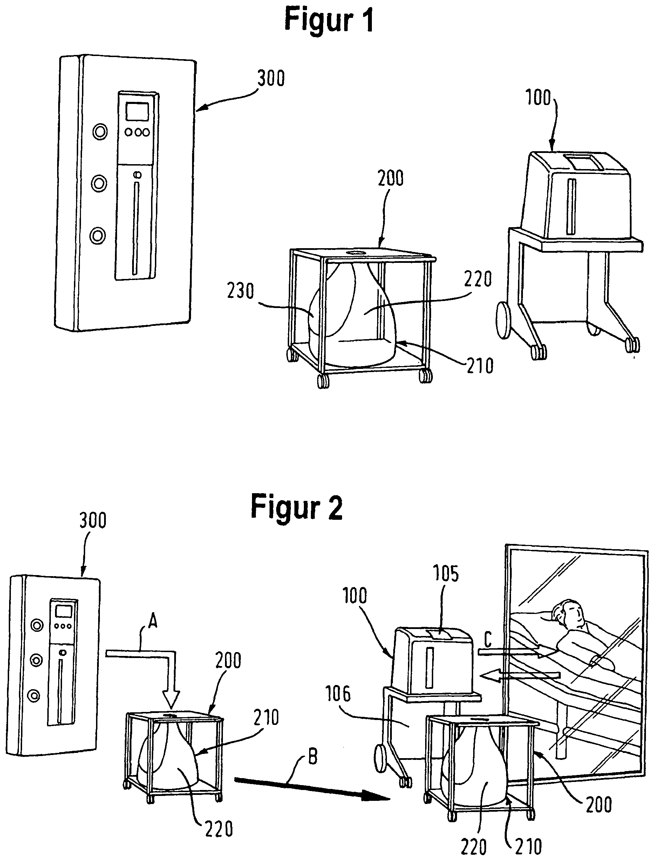

Figur 1 zeigt ein System zur Durchführung einer Blutbehandlung gemäß der vorliegenden Erfindung. Es umfasst das eigentliche Blutbehandlungsgerät 100 mit Mitteln zur externen Blutbehandlung, die mobile Vorrichtung 200 sowie die Befülleinheit 300.

Vorzugsweise dient das System zur extrakorporalen Blutbehandlung, insbesondere für die Hämodialyse, die Home-Hämodialyse (HHD) und die Behandlung bei akutem Nierenversagen. Andere Anwendungsgebiete können auch die Peritonealdialyse, insbesondere die APD sein. Das vorliegende System kann für diskontinuierliche Verfahren (iHD und EDD) sowie auch für kontinuierliche Anwendungen (beispielsweise CWHD und CWH) verwendet werden.

Die mobile Vorrichtung 200 weist in Form eines Doppelkammerbeutels 210 (oder in Form zweier separater Beutel) einen Tank auf, dessen eine Kammer mit frischer Dialysierflüssigkeit 220 befüllt wird, die ihrerseits mit Hilfe der Befülleinheit 300 hergestellt und in den Doppelkammerbeutel 210 eingefüllt wird. In dem hier dargestellten Ausführungsbeispiel wird die Dialysierflüssigkeit somit in Form eines Batches bereitgestellt.

Von der Erfindung ist jedoch beispielsweise auch der Fall umfasst, dass Dialysierflüssigkeiten, die beispielsweise in Beuteln oder sonstigen Aufnahmebehältnissen vorliegen, genutzt werden können und mit einer mobilen Vorrichtung zu dem Blutbehandlungsgerät 100 verfahren werden. Dabei kann es sich beispielsweise auch um HF-Lösungen handeln.

Der Batch an Dialysierflüssigkeit wird lokal durch die vorzugsweise ortsfeste Befülleinheit 300 durch die Verdünnung von Konzentraten mit gereinigtem Wasser, vorzugsweise mit RO-Wasser hergestellt. Bei der Befülleinheit 300 kann es sich um eine kleine beispielsweise an einer Wand zu befestigende oder anderweitig ortsfeste oder auch bewegliche Einheit handeln. Sie kann eine separate RO-Einheit aufweisen bzw. mit dieser in Verbindung stehen oder auch eine integrierte RO-Einheit enthalten. Auch weitere Wasserbehandlungseinheiten können in der Befülleinheit angeordnet sein. Dazu gehören beispielsweise Mittel zur Zuführung von Desinfektionsmitteln, die gegebenenfalls zur Desinfektion von Komponenten bzw. des für die Herstellung der Dialysierflüssigkeit verwendeten Wassers herangezogen werden. Vorteilhaft ist es weiter, dass die Befülleinheit Heizmittel aufweist, mit denen das Wasser bzw. die hergestellte Dialysierflüssigkeit aufgewärmt werden kann.

Zur Herstellung der Dialysierflüssigkeit können flüssige Konzentrate oder auch feste Konzentrate verwendet werden, die sodann mit dem Wasser gemischt werden, um die Dialysierflüssigkeit herzustellen.

Vorzugsweise werden in der Befülleinheit 300 Verbindungen mit minimalem (reduzierten) Kontaminationsrisiko verwendet, um sicherzustellen, dass die Herstellung der Dialysierflüssigkeit unter keimarmen Bedingungen erfolgt.

Nach der Herstellung der Dialysierflüssigkeit 220 wird diese in den Tank 210 bzw. in eine Kammer des Doppelkammerbeutels 210 der mobilen Vorrichtung 200 eingefüllt. Dieser Vorgang ist schematisch in Figur 2 dargestellt und mit dem Bezugszeichen A gekennzeichnet. Der Beutel 210 ist in der mobilen Vorrichtung 200 zu Transportzwecken angeordnet. Die Größe des Beutels bzw. des Tanks 210 kann beispielsweise im Bereich 15 I bis 120 I, vorzugsweise im Bereich von 40 I bis 80 I, liegen.

Der Beutel 210 der mobilen Vorrichtung 200 kann vorzugsweise flexibel sein und die beiden Kammern des Doppelkammerbeutels 210 stehen derart miteinander in Verbindung, dass die Volumenvergrößerung einer Kammer zu einer entsprechenden Volumenverkleinerung der anderen Kammer führen kann, wie dies unten noch näher dargestellt wird.

Nach der Befüllung des Tankes 210 der mobilen Vorrichtung 200 wird diese zu dem Blutbehandlungsgerät 100 verfahren (Schritt B), wozu die mobile Vorrichtung 200 mit Rollen ausgeführt ist.

Das Blutbehandlungsgerät 100 kann eine Steuereinheit umfassen, die erkennt, wenn die mobile Vorrichtung 200 ihre vordefinierte Position (dabei kann es sich beispielsweise um die Endposition oder auch um Zwischenpositonen handeln) in dem Blutbehandlungsgerät 100 erreicht hat. Das Blutbehandlungsgerät 100 umfasst des weiteren eine Nutzerschnittstelle, mittels derer dem Nutzer Betriebszustände des Blutbehandlungsgeräts 100 bzw. der mobilen Vorrichtung 200 angezeigt werden können und mittels derer der Nutzer gegebenenfalls Eingaben machen kann. Das Blutbehandlungsgerät 100 weist des weiteren Mittel zur Durchführung einer extrakorporalen Blutbehandlung, insbesondere Mittel zur Durchführung einer Dialysebehandlung, wie beispielsweise ein Schlauchset (Kassettensystem/integrierter Einmalartikel), eine Blutpumpe, eine Dialysatpumpe, einen Dialysator mit entsprechenden Schlauchleitungen, etc. auf.

Nachdem die mobile Vorrichtung 200 ihre vorgesehene Position in dem Blutbehandlungsgerät 100 erreicht hat bzw. in dieses integriert ist, wird automatisch oder auf Aufforderung durch den Nutzer eine Fluidverbindung zwischen dem Tank 210 der mobilen Vorrichtung 200 und der dem Blutbehandlungsgerät 100 hergestellt. Diese Fluidverbindung ist so ausgeführt, dass frische Behandlungsflüssigkeit 220 aus einem Teil des Doppelkammerbeutels 210 dem Blutbehandlungsgerät 100 zugeführt wird, während die Dialyse läuft. Eine weitere Fluidverbindung ist so ausgeführt, dass während der Dialysebehandlung anfallende, gebrauchte Dialysierflüssigkeit 230 in die andere Kammer des Doppelkammerbeutels 210 eingefüllt wird. Zusätzlich kann in diese Kammer oder auch in eine weitere Kammer Ultrafiltrat abgeführt werden. Das Blutbehandlungsgerät besteht aus der eigentlichen Behandlungseinheit 105, unter der ein Aufnahmeraum 106 zur Aufnahme der mobilen Vorrichtung angeordnet ist.

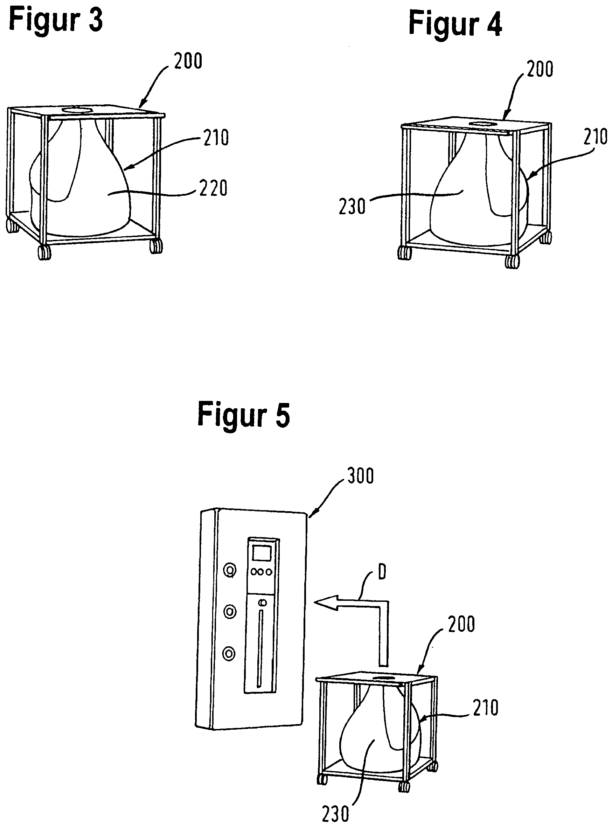

Figur 2 zeigt den Prozess des Ankoppelns der mobilen Vorrichtung 200 an das Blutbehandlungsgerät 100. Durch die Doppelpfeile in Figur 3 ist der extrakorporale Blutkreislauf gemäß Schritt C symbolisiert, über den der Patient mit dem Blutbehandlungsgerät 100 in Verbindung steht.

Während der Dialysebehandlung bzw. Blutbehandlung kann die mobile Vorrichtung 200 erforderlichenfalls ausgetauscht werden ohne dass der Patient von dem extrakorporalen Blutkreislauf diskonnektiert werden müßte. Die mobile Vorrichtung 200 kann dann durch eine mobile Vorrichtung 200 ausgetauscht werden, die einen mit frischer Behandlungsflüssigkeit 220 gefüllten Tank 210 aufweist.

Figur 4 zeigt den Tank 210 der mobilen Vorrichtung 200 in einem Zustand, in dem die Kammer zur Aufnahme der verbrauchten Behandlungsflüssigkeit 230 leer ist und die Kammer zur Aufnahme der frischen Dialysierflüssigkeit 220 gefüllt ist. Während der Blutbehandlung sinkt das Volumen der Kammer, in der die frische Dialysierflüssigkeit 220 vorliegt. Gleichzeitig erhöht sich durch die Rückführung verbrauchter Dialysierflüssigkeit das Volumen der Kammer, die zur Aufnahme der verbrauchten Dialysierflüssigkeit und gegebenenfalls von Ultrafiltrat dient.

Figur 4 zeigt den Doppelkammerbeutel 210 der mobilen Vorrichtung 200 in einem Zustand nach der Behandlung. Hier ist ersichtlich, dass nun die Kammer des Doppelkammerbeutels 210 gefüllt ist, die zur Aufnahme der verbrauchten Dialysierflüssigkeit 230 und gegebenenfalls von Ultrafiltrat dient.

Wird kein Ultrafiltrat abgezogen, ändert sich während des Vorgangs das Volumen des Beutels 210 nicht oder kaum, da in dem Maße, wie frische Dialysierflüssigkeit 220 abgezogen wird, verbrauchte Dialysierflüssigkeit 230 zugeführt wird.

Selbstverständlich sind von der Erfindung auch andere Ausführungsformen des Tanks 210 umfasst. Der Tank kann beispielsweise starr ausgeführt sein. Auch ist von der Erfindung der Fall umfasst, dass voneinander getrennte, das heißt nicht von miteinander in Verbindung stehende Beutel oder sonstige Gefäße für die unverbrauchte Dialysierflüssigkeit 220 einerseits und für die gebrauchte Dialysierflüssigkeit 230 andererseits verwendet werden.

Nachdem die Dialysierflüssigkeit gebraucht wurde, wird die mobile Vorrichtung 200 von dem Blutbehandlungsgerät 100 abgekoppelt und zu der Befülleinheit 300 verfahren. Hier wird die verbrauchte Dialysierflüssigkeit 230 und gegebenenfalls Ultrafiltrat aus dem Beutel 210 durch Pumpen abgezogen und sodann der als Einmalartikel verwendete Tank bzw. Beutel 210 verworfen.

Die mobile Vorrichtung 200 kann sodann mit einem neuen Tank bzw. Beutel 210 verwendet werden und steht dann wieder zur Befüllung zur Verfügung.

Den Vorgang der Entleerung des Beutels 210 der mobilen Vorrichtung 200 durch die Befülleinheit 300 ist in Figur 5 (Schnitt D) dargestellt.

Die Entleerung erfolgt z.B. durch eine Pumpe der Befülleinheit 300, die in diesem Fall auch als Entleerungseinheit dient. Grundsätzlich ist es ebenfalls denkbar, für die Befüllung und Entleerung unterschiedliche Einheiten zu verwenden oder eine separate (einzelne) Pumpe.

Das Blutbehandlungsgerät 100 kann mit Steuerungsmitteln derart ausgeführt sein, dass dessen Bedienung besonders einfach ist. So ist es beispielsweise denkbar, dass automatisch erkannt wird, wenn eine mobile Vorrichtung 200 in das Blutbehandlungsgerät 100 eingeführt ist. Dann kann automatisch eine Konnektion mit dem Tank 210 bzw. mit dem Beutel 210 der mobilen Vorrichtung 200 erfolgen. Darüber hinaus kann eine Datenverbindung dahingehend erfolgen, dass Daten von der mobilen Vorrichtung 200 durch das Blutbehandlungsgerät 100 ausgelesen werden. Diese Daten können beispielsweise die in dem Beutel 210 befindliche Dialysierflüssigkeit 220 (Menge, Zusammensetzung) betreffen.

Denkbar ist, es die erfolgreiche Konnektion dem Nutzer anzuzeigen und diesen durch Betätigung eines weiteren Tastenfeldes oder eines Schalters oder dergleichen die Dialysebehandlung zu starten.

Ebenso kann vorgesehen sein, dass das Blutbehandlungsgerät 100 dem Nutzer anzeigt, wenn die Dialysebehandlung beendet ist oder wenn die Aufnahmekapazität des Beutels 210 für verbrauchte Dialysierflüssigkeit 230 erschöpft ist.

In diesem Fall kann dem Nutzer beispielsweise akustisch und/oder optisch angezeigt werden, dass die mobile Vorrichtung 200 von dem Blutbehandlungsgerät 100 diskonnektiert werden muss.

In dem hier dargestellten Ausführungsbeispiel weist das Blutbehandlungsgerät 100 Mittel zur Durchführung der extrakorporalen Blutbehandlung auf. Einzige Ausnahme ist ein Tank zur Aufnahme frischer Dialysierflüssigkeit 220. Ein solcher Tank ist in dem Blutbehandlungsgerät 100 nicht vorgesehen sondern wird, wie oben ausgeführt, durch eine mobile Vorrichtung 200 bereitgestellt, die zu dem Blutbehandlungsgerät 100 verfahren werden kann, um die Dialyse beginnen zu können.

Die Vorteile des Systems lassen sich in einer bevorzugten Ausgestaltung der Erfindung wie folgt zusammenfassen:
Batchwechsel während der Patient angeschlossen bleibt
Kein Beutelwechsel (5 l Bags / Waste Bag)
Reduzierter Arbeitsaufwand (Wasteentsorgung mit mobiler Einheit)
Hygiene: geschlossenes System, reduzierte Kontaminationsgefahr
Einmalartikel basierte Fluidik (keine feste Hydraulik im mobilen System/Behandlungseinheit, reduzierter Desinfektionsaufwand)
Integriertes Alarmmanagement (Informationstransfer)

## Patentansprüche

1. System zur Durchführung einer Dialysebehandlung, umfassend:
ein Blutbehandlungsgerät (100), insbesondere ein Dialysegerät (100), und
einen Tank (210), aus dem während der Durchführung der Blutbehandlung Dialysierflüssigkeit (220) entnommen wird und in den während der Durchführung der Blutbehandlung verbrauchte Dialysierflüssigkeit (230) eingefüllt wird, wobei
der Tank (210) Bestandteil einer mobilen Vorrichtung (200) ist, die mit dem Blutbehandlungsgerät (100) derart verbindbar ist, dass eine Fluidverbindung zwischen dem Tank (210) und dem Blutbehandlungsgerät (100) herstellbar ist, und
das System des Weiteren eine Befülleinheit (300) aufweist, mittels derer der Tank (210) der mobilen Vorrichtung (100) mit Dialysierflüssigkeit (220), befüllbar ist
**dadurch gekennzeichnet, dass**
der Tank (210) in Form von mehreren einzelnen Tanks ausgeführt ist, von denen ein erster Tank zur Abgabe von Dialysierflüssigkeit (220) und ein zweiter Tank zur Aufnahme verbrauchter Dialysierflüssigkeit (230), dient, wobei
der zweite Tank für das verbrauchte Dialysat den ersten Tank für das frische Dialysat umschließt.

2. System nach Anspruch 1, **dadurch gekennzeichnet, dass** das Blutbehandlungsgerät (100) selbst keinen Tank aufweist, aus dem während der der Blutbehandlung Dialysierflüssigkeit (220) entnommen wird und/oder in den während der Durchführung der Blutbehandlung verbrauchte Dialysierflüssigkeit (230) eingefüllt wird.

3. System nach Anspruch 1 oder 2, **dadurch gekennzeichnet, dass** das System Mittel aufweist, die derart ausgeführt sind, dass sie nach dem Bewegen der mobilen Vorrichtung (200) in das Blutbehandlungsgerät (100) in eine vorbestimmte Position automatisch oder auf Aufforderung wenigstens eine Fluidverbindung zwischen dem Tank (210) und dem Blutbehandlungsgerät (100) herstellen.

4. System nach einem der vorhergehenden Ansprüche, **dadurch gekennzeichnet, dass** das Blutbehandlungsgerät (100) eine Aufnahme, vorzugsweise eine Ausnehmung oder einen Raum zur Aufnahme der mobilen Vorrichtung (200) aufweist.

5. System nach einem der vorhergehenden Ansprüche, **dadurch gekennzeichnet, dass** die mobile Vorrichtung (200) zum Zwecke der leichten Bewegbarkeit Rollen aufweist.

6. System nach einem der vorhergehenden Ansprüche, **dadurch gekennzeichnet, dass** der Tank (210) als starres Gefäß oder als Beutel (210) mit flexiblen Wandungen ausgeführt ist.

7. System nach einem der vorhergehenden Ansprüche, **dadurch gekennzeichnet, dass** die mobile Vorrichtung (200) derart ausgeführt ist, dass sie den Tank (210), insbesondere den Beutel (210) mechanisch stützt.

8. System nach einem der vorhergehenden Ansprüche, **dadurch gekennzeichnet, dass** die mobile Vorrichtung (200) gasdicht ausgeführt ist.

9. System nach einem der vorhergehenden Ansprüche, **dadurch gekennzeichnet, dass** die mobile Vorrichtung (200) Mittel zum Ableiten verbrauchter Dialysierflüssigkeit (230) in einen Abfluß oder in eine sonstige Aufnahmeeinheit aufweist.

10. System nach einem der vorhergehenden Ansprüche, **dadurch gekennzeichnet, dass** die Befülleinheit (300) Mittel aufweist, mittels derer die Dialysierflüssigkeit (220) aus mehreren Komponenten, vorzugsweise aus einem oder mehreren Konzentraten und Wasser herstellbar ist.

11. System nach einem der vorhergehenden Ansprüche, **dadurch gekennzeichnet, dass** die Befülleinheit (300) mit einer RO-Wasserversorgungseinheit in Verbindung steht oder eine solche Einheit aufweist.

12. Verfahren zum Betätigen des Systems nach einem der vorhergehenden Ansprüche, nämlich zum Versorgen des Blutbehandlungsgerätes (100), insbesondere eines Dialysegerätes (100), mit der Dialysierflüssigkeit (220), die während der Durchführung der Blutbehandlung aus dem Tank (210) entnommen wird, wobei der Tank (210) Bestandteil der mobilen Vorrichtung (200) ist, die zur Durchführung der Blutbehandlung mit dem Blutbehandlungsgerät (100) derart verbunden wird, dass eine Fluidverbindung zwischen dem Tank (210) und dem Blutbehandlungsgerät (100) besteht, wobei die mobile Vorrichtung (200) zunächst zu der Befülleinheit (300) bewegt wird, in oder an der der Tank (210) der mobilen Vorrichtung (200) mit Dialysierflüssigkeit (220) befüllt wird.

13. Verfahren nach Anspruch 12, **dadurch gekennzeichnet, dass** das Verfahren des Weiteren den Schritt umfaßt, dass verbrauchte Dialysierflüssigkeit (230), insbesondere verbrauchte Dialysierflüssigkeit (230), dem bzw. einem Tank (210) der mobilen Vorrichtung (200) zugeführt wird oder mittels der mobilen Vorrichtung (200) oder direkt ohne die mobile Vorrichtung (200) einem Ablauf oder einer sonstigen Aufnahmeeinheit zugeführt wird.

14. Verfahren nach einem der Ansprüche 12 oder 13, **dadurch gekennzeichnet, dass** das Verfahren derart durchgeführt wird, dass dem wenigstens einen Tank (210) gleichzeitig oder zeitversetzt Dialysierflüssigkeit (220) entnommen und verbrauchte Dialysierflüssigkeit (230) zugeführt wird.

15. Verfahren nach einem der Ansprüche 12 bis 14, **dadurch gekennzeichnet, dass** nach dem Bewegen der mobilen Vorrichtung (200) zu dem Blutbehandlungsgerät (100) in eine vorgegebene Position automatisch oder auf Aufforderung wenigstens eine Fluidverbindung zwischen dem Tank (210) und dem Blutbehandlungsgerät (100) hergestellt wird.

16. Verfahren nach einem der Ansprüche 12 bis 15, **dadurch gekennzeichnet, dass** nach dem Bewegen der mobilen Vorrichtung (200) zu dem Blutbehandlungsgerät (100) in eine vorgegebene Position automatisch oder auf Aufforderung wenigstens eine Datenverbindung zwischen der mobilen Vorrichtung (200) und dem Blutbehandlungsgerät (100) hergestellt wird.

17. Verfahren nach einem der Ansprüche 12 bis 16, **dadurch gekennzeichnet, dass** die mobile Vorrichtung (200) zu der Befülleinheit (300) oder zu einer separaten Entleerungseinheit verfahren wird und verbrauchte Dialysierflüssigkeit (230), insbesondere verbrauchte Dialysierflüssigkeit (230), aus dem Tank (210) der mobilen Vorrichtung (200) mittels der Befülleinheit (300) oder mittels der separaten Entleerungseinheit entsorgt wird.

## Claims

1. A system for performing a dialysis treatment, the system including:
a blood treatment device (100), in particular a dialyzer (100), and
a tank (210), from which dialyzing fluid (220) is taken while performing the blood treatment, and into which dialyzing fluid (230) consumed during the performance of the blood treatment is filled, wherein
the tank (210) forms part of a mobile device (200), which can be connected to the blood treatment device (100) in such a way that a fluidic connection can be established between the tank (210) and the blood treatment device (100), and
the system further comprises a filling unit (300), by means of which the tank (210) of the mobile device (100) can be filled with dialyzing fluid (220),
**characterized in that**
the tank (210) is configured in the form of multiple individual tanks, from which a first tank serves to dispense dialyzing fluid (220) and a second tank serves to receive consumed dialyzing fluid (230), wherein
the second tank for the consumed dialysate encloses the first tank for the fresh dialysate.

2. The system according to claim 1, **characterized in that** the blood treatment device (100) does *per se* not comprise a tank from which dialyzing fluid (220) is taken during the blood treatment and/or in which consumed dialyzing fluid (230) is filled during the performance of the blood treatment.

3. The system according to claim 1 or 2, **characterized in that** the system comprises means, which are configured in such a way, that after displacement of the mobile device (200) into the blood treatment device (100) in a predetermined position, they establish at least one fluidic connection between the tank (210) and the blood treatment device (100).

4. The system according to one of the preceding claims, **characterized in that** the blood treatment device (100) comprises a receptacle, preferably a recess or a space for receiving the mobile device (200).

5. The system according to one of the preceding claims, **characterized in that** the mobile device (200) comprises rollers for the purpose of easier movability.

6. The system according to one of the preceding claims, **characterized in that** the tank (210) is formed as a rigid vessel or as a bag (210) with flexible walls.

7. The system according to one of the preceding claims, **characterized in that** the mobile device (200) is formed in such a way that it mechanically supports the tank (210), in particular the bag (210).

8. The system according to one of the preceding claims, **characterized in that** the mobile device (200) is formed to be gas-tight.

9. The system according to one of the preceding claims, **characterized in that** the mobile device (200) comprises means (200) for the discharging of consumed dialyzing fluid (230) into a drain or another receiving unit.

10. The system according to one of the preceding claims, **characterized in that** the filling unit (200) comprises means, by means of which the dialyzing fluid (220) can be produced from multiple components, preferably of one or more concentrates and water.

11. The system according to one of the preceding claims, **characterized in that** the filling unit (300) is connected to a RO water supply unit or comprises such a unit.

12. The method for actuating the system according to one of the preceding claims, that is for the supply of a blood treatment device (100), in particular a dialysis device (110), with the dialyzing fluid (220), which is taken from the tank (210) while performing the blood treatment, wherein the tank (210) is a part of the device (200), which, in order to perform the blood treatment, is connected to the blood treatment device (100) in such a way that there is a fluidic connection between the tank (210) and the blood treatment device (100), wherein the mobile device (200) is displaced towards the filling unit (300) first, in which or on which the tank (210) of the mobile device (200) is filled with dialyzing fluid (220).

13. The method according to claim 12, **characterized in that** the method further comprises the step of feeding consumed dialyzing fluid (230), in particular consumed dialyzing fluid (230), is fed to a or the tank (210) of the mobile device (200), or is fed to a drain or another receiving unit by means of the mobile device (200) or directly without the mobile device (200).

14. The method according to one of claims 12 or 13, **characterized in that** the method is performed in such a way, that the at least one tank (210) has dialyzing fluid (220) fed to it or consumed dialyzing fluid (230) taken from it at the same time or offset in time-.

15. The method according to one of claims 12 to 14, **characterized in that** at least one fluidic connection is established between the tank (210) and the blood treatment device (100) after displacement of the mobile device (200) towards the blood treatment device (100) into a predetermined position.

16. The method according to one of claims 12 to 15, **characterized in that** after automatic or requested displacement of the mobile device (200) towards the blood treatment device (100) into a predetermined position, at least one data connection is established between the mobile device (200) and the blood treatment device (100).

17. The method according to one of claims 12 to 16, **characterized in that** the mobile device (200) is displaced towards the filling unit (300) or to a separate emptying unit and consumed dialyzing fluid (230), in particular consumed dialyzing fluid (230), is discharged from the tank (210) of the mobile device (300) by means of the filling unit (300) or by means of the separate emptying unit.

## Revendications

1. Système destiné à la réalisation d'un traitement de dialyse, comprenant
un appareil de traitement du sang (100), notamment un appareil de dialyse (100), et
un réservoir (210) hors duquel du liquide de dialyse (220) est prélevé pendant la réalisation du traitement du sang et dans lequel le liquide de dialyse souillé (230) est rempli pendant la réalisation du traitement du sang,
le réservoir (210) faisant partie intégrante d'un dispositif mobile (200) qui est raccordable à l'appareil de traitement du sang (100) de manière à ce qu'une liaison fluidique entre le réservoir (210) et l'appareil de traitement du sang (100) puisse être établie, et
le système présentant en outre une unité de remplissage (300) au moyen de laquelle le réservoir (210) du dispositif mobile (100) peut être rempli avec du liquide de dialyse (220),
**caractérisé en ce que**
le réservoir (201) est réalisé sous forme de plusieurs réservoirs individuels dont un premier réservoir sert à la décharge du liquide de dialyse (220) et un deuxième réservoir sert à réceptionner le liquide de dialyse souillé (230),
le deuxième réservoir pour le dialysat souillé entourant le premier réservoir pour le dialysat frais.

2. Système selon la revendication 1, **caractérisé en ce que** l'appareil de traitement de sang (100) ne présente lui-même pas de réservoir hors duquel du liquide de dialyse (220) est prélevé pendant le traitement du sang et/ou dans lequel du liquide de dialyse souillé (230) est rempli pendant la réalisation du traitement du sang,

3. Système selon la revendication 1 ou 2, **caractérisé en ce que** le système présente des moyens qui sont réalisés de manière à ce qu'après le déplacement du dispositif mobile (200) dans l'appareil de traitement du sang (100) dans une position prédéterminée, ils établissement automatiquement ou sur demande au moins une liaison fluidique entre le réservoir (210) et l'appareil de traitement du sang (100).

4. Système selon l'une quelconque des revendications précédentes, **caractérisé en ce que** l'appareil de traitement du sang (100) présente un logement, de préférence un évidement ou un espace destiné à loger le dispositif mobile (200).

5. Système selon l'une quelconque des revendications précédentes, **caractérisé en ce que** le dispositif mobile (200) présente des roulettes à des fins de facilité de déplacement.

6. Système selon l'une quelconque des revendications précédentes, **caractérisé en ce que** le réservoir (210) est réalisé comme récipient rigide ou comme sachet (210) à parois flexibles.

7. Système selon l'une quelconque des revendications précédentes, **caractérisé en ce que** le dispositif mobile (200) est réalisé de manière à ce qu'il supporte le réservoir (210) mécaniquement, notamment le sachet (210).

8. Système selon l'une quelconque des revendications précédentes, **caractérisé en ce que** le dispositif mobile (200) est réalisé de manière étanche aux gaz.

9. Système selon l'une quelconque des revendications précédentes, **caractérisé en ce que** le dispositif mobile (200) présente des moyens d'évacuation du liquide de dialyse souillé (230) dans un égout ou dans une autre unité de réception.

10. Système selon l'une quelconque des revendications précédentes, **caractérisé en ce que** l'unité de remplissage (300) présente des moyens au moyen desquels le liquide de dialyse (220) peut être produite à partir de plusieurs composants, de préférence à partir d'un ou de plusieurs concentrés et d'eau.

11. Système selon l'une quelconque des revendications précédentes, **caractérisé en ce que** l'unité de remplissage (300) est en liaison avec une unité d'alimentation en eau à osmose inverse ou présente une telle unité.

12. Procédé d'actionnement du système selon l'une quelconque des revendications précédentes, à savoir pour l'alimentation de l'appareil de traitement du sang (100), notamment d'un appareil de dialyse (100), avec le liquide de dialyse (220) qui est prélevé du réservoir (210) pendant la réalisation du traitement du sang, le réservoir (210) faisant partie intégrante du dispositif mobile (200) qui, pour la réalisation du traitement du sang, est relié à l'appareil de traitement du sang (100) de manière à ce qu'il y ait une liaison fluidique entre le réservoir (210) et l'appareil de traitement du sang (100),
le dispositif mobile (200) étant d'abord déplacé vers l'unité de remplissage (300), étant rempli avec du liquide de dialyse (220) dans ou sur le réservoir (210) du dispositif mobile (200).

13. Procédé selon la revendication 12, **caractérisé en ce que** le procédé comprend en outre l'étape consistant **en ce que** le liquide de dialyse souillé (230), notamment du liquide de dialyse souillé (230) est amené au réservoir ou à un réservoir (210) du dispositif mobile (200) ou est amené à un égout ou à une autre unité de réception au moyen du dispositif mobile (200) ou directement sans le dispositif mobile (200).

14. Procédé selon l'une quelconque des revendications 12 ou 13, **caractérisé en ce que** le procédé est réalisé de manière à ce que, simultanément ou de manière temporellement décalée, du liquide de dialyse (220) est prélevé à l'au moins un réservoir (210) ou **en ce que** du liquide de dialyse souillé (230) est amené à l'au moins un réservoir (210).

15. Procédé selon l'une quelconque des revendications 12 à 14, **caractérisé en ce qu'**après le déplacement du dispositif mobile (200) vers l'appareil de traitement du sang (100) dans une position prédéterminée, au moins une liaison fluidique entre le réservoir (210) et l'appareil de traitement de sang (100) est établie automatiquement ou sur demande.

16. Procédé selon l'une quelconque des revendications 12 à 15, **caractérisé en ce qu'**après le déplacement du dispositif mobile (200) vers l'appareil de traitement du sang (100) dans une position prédéterminée, au moins une liaison de données entre le dispositif mobile (200) et l'appareil de traitement du sang (100) est établie automatiquement ou sur demande.

17. Procédé selon l'une quelconque des revendications 12 à 16, **caractérisé en ce que** le dispositif mobile (200) est déplacé vers l'unité de remplissage (300) ou vers une unité de vidange séparée et **en ce que** du liquide de dialyse souillé (230), notamment du liquide de dialyse souillé (230), est éliminé hors du réservoir (210) du dispositif mobile (200) au moyen de l'unité de remplissage (300) ou au moyen de l'unité de vidange séparée.
